(19) **Europäisches Patentamt**
**European Patent Office**
**Office européen des brevets**

(11) **EP 4 294 506 B1**

(12) **EUROPEAN PATENT SPECIFICATION**

(45) Date of publication and mention
of the grant of the patent:
**10.09.2025 Bulletin 2025/37**

(51) International Patent Classification (IPC):
*A61N 1/37* (2006.01)     *A61B 5/349* (2021.01)
*A61B 5/29* (2021.01)     *A61N 1/365* (2006.01)
*A61N 1/375* (2006.01)     *A61B 5/308* (2021.01)
*A61B 5/00* (2006.01)

(21) Application number: **22705750.2**

(22) Date of filing: **07.02.2022**

(52) Cooperative Patent Classification (CPC):
**A61N 1/3704; A61B 5/29; A61B 5/349;**
A61B 5/0031; A61B 5/308; A61B 5/353;
A61N 1/36507; A61N 1/3756

(86) International application number:
**PCT/EP2022/052813**

(87) International publication number:
**WO 2022/175123 (25.08.2022 Gazette 2022/34)**

(54) **AN IMPLANTABLE MEDICAL DEVICE CONFIGURED TO PROVIDE AN INTRACARDIAC FUNCTION**

IMPLANTIERBARE MEDIZINISCHE VORRICHTUNG ZUR BEREITSTELLUNG EINER INTRAKARDIALEN FUNKTION

DISPOSITIF MÉDICAL IMPLANTABLE CONÇU POUR FOURNIR UNE FONCTION INTRACARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2021   US 202163150108 P**
**05.03.2021   EP 21161019**

(43) Date of publication of application:
**27.12.2023 Bulletin 2023/52**

(73) Proprietor: **BIOTRONIK SE & Co. KG**
**12359 Berlin (DE)**

(72) Inventors:
• **MIDGETT, Madeline Anne**
**Portland, Oregon 97213 (US)**
• **YOUNG, Daniel**
**Portland, Oregon 97202 (US)**
• **JONES, Christopher**
**Oregon City, Oregon 97045 (US)**
• **WHITTINGTON, R. Hollis**
**Portland, Oregon 97202 (US)**

(74) Representative: **Biotronik Corporate Services SE**
**Corporate Intellectual Property**
**Sieversufer 7-9**
**12359 Berlin (DE)**

(56) References cited:
**EP-A1- 2 108 400      US-A- 5 339 820**
**US-A- 5 755 738      US-A1- 2008 262 559**
**US-A1- 2013 325 081**

**Description**

The present invention generally relates to an implantable medical device for providing an intracardiac function, in particular a pacing function such as ventricular pacing, specifically VDD pacing.

In an implantable medical device, e.g. in the shape of a leadless pacemaker device or a cardiac stimulation device using a subcutaneously implanted pulse generator and one or multiple leads extending into a patient's heart, it may be desirous to provide stimulation in a ventricle of the patient's heart, e.g. in the right ventricle, in synchrony with an atrial activity. For this, ventricular pacing shall take into account atrial sense signals to control the ventricular pacing based on atrial events indicative of atrial activity, for example in a so-called VDD pacing mode.

In recent years, leadless pacemakers have received increasing attention. Leadless pacemakers, in contrast to pacemakers implanted subcutaneously using leads extending transvenously into the heart, avoid leads in that the pacemaker device itself is implanted into the heart, the pacemaker having the shape of a capsule for implantation into cardiac tissue, in particular the the right ventricle. Such leadless pacemakers exhibit the inherent advantage of not using leads, which can reduce risks for the patient involved with leads transvenously accessing the heart, such as the risk of pneumothorax, lead dislodgement, cardiac perforation, venous thrombosis and the like.

A leadless pacemaker or a lead of a stimulation device may specifically be designed for implantation in the right ventricle and, in this case, during implant is placed e.g. in the vicinity of the apex of the right ventricle. Ventricular pacing may for example be indicated in case a dysfunction at the AV node occurs, but the sinus node function is intact and appropriate. In such a case in particular a so-called VDD pacing may be desired, involving ventricular pacing with atrial tracking and hence requiring sensing of atrial activity in order to a pace at the ventricle based on intrinsic atrial contractions.

A VDD pacing is in particular motivated by patient hemodynamic benefits of atrioventricular (AV) synchrony by utilizing an appropriate sinus node function to trigger ventricular pacing, potentially allowing to maximize ventricular preload, to limit AV valve regurgitation, to maintain low mean atrial pressure, and to regulate autonomic and neurohumoral reflexes.

Publications have explored solutions to use modalities to detect mechanical events of atrial contractions, including the sensing of motion, sound and pressure (see for example US 2018/0021581 A1 disclosing a leadless cardiac pacemaker including a pressure sensor and/or an accelerometer to determine an atrial contraction timing). As mechanical events generally exhibit a small signal volume, signal detection based on mechanical events, for example motion, sound or pressure, may be difficult to sense, in particular when the implantable medical device is placed in the ventricle and hence rather far removed from the atrium of which contractions shall be sensed. In addition, wall motion and movement of blood generated by atrial contractions may not be directly translated to the ventricle, and cardiac hemodynamic signals, such as motion, heart sounds and pressure, are likely affected by external factors such as posture and patient activity.

US 2008/0262559 A1 discloses a method and an apparatus for automatically adjusting the sensing threshold of cardiac rhythm management devices. The invention is particularly suited for implementation in devices such as implantable cardiac pacemakers and implantable cardioverter/defibrillators. A method and apparatus are provided in which a noise level and signal level for a sensing channel are determined for each cardiac cycle with the sensing threshold of the channel being adjusted in accordance therewith.

EP 2 108 400 A1 discloses a system for automatically adjusting a sensing threshold in a cardiac rhythm management device, the system comprising means for increasing an atrial sensing threshold during a time period in response to a ventricular depolarization or the delivery of a ventricular pace puls and/or means for increasing a ventricular sensing threshold during a time period in response to an atrial depolarization or the delivery of an atrial pace pulse

It is an objective to provide an implantable medical device and a method for operating an implantable medical device allowing, in particular, for ventricular pacing with atrioventricular synchrony, hence requiring a reliable sensing of atrial events in order to provide for a ventricular pacing based on such atrial events.

Such desires are addressed by an implantable medical device configured to provide for an intracardiac function having the features of claim 1 and a method for operating an implantable medical device for providing for an intracardiac function having the features of claim 14.

The present invention is defined by the appended claims.

The sense threshold generally is used to detect atrial events. Generally, if a cardiac sense signal associated with atrial activity crosses the sense threshold, it is assumed that an atrial event is present. Herein, a processing scheme may be employed, for example a windowing scheme, to differentiate signal portions relating to atrial activity in the far field from signal portions relating to ventricular activity in the near field, assuming for example that the implantable medical device is implanted in a ventricle, for example the right ventricle of the patient's heart.

In order to reliably detect atrial activity, a fixed sense threshold may not be sufficient, as sense signals relating to atrial activity may vary. Herein, if atrial events are missed - i.e., for one or multiple cardiac cycles no atrial events are detected as for example no threshold crossing of a corresponding sense signal is identified - the sensing threshold may be adjusted such that atrial activity is recaptured, hence allowing for a reliable, steady sensing action for example in the context of atrial-ventricular synchronous pacing.

For this reason, it herein is proposed to use an approach in which the sense threshold is dynamically adjusted, in particular reduced, based on one or multiple reduction criteria, which take into account missed atrial events in a number of cardiac cycles.

The reduction criterion may be defined as a short-term criterion or a long-term criterion.

Within a short-term criterion it is checked whether in very recent cardiac cycles atrial events have been missed. For the short-term criterion Y1 may for example have a value in between 1 to 4, for example 2. X1 may have for example also a value between 1 to Y1. For example, within the reduction criterion it may be checked whether one out of two cardiac cycles immediately prior to the current cardiac cycle has been missed, or it may be checked whether two out of two cardiac cycles immediately prior to the current cardiac cycle have been missed.

Within a long-term criterion it may for example be checked whether, over an increased number of cardiac cycles, a significant number of atrial events have been missed. For the long-term criterion Y1 may for example have a value between 4 and 16, for example 8. X1 may have a value of for example 3 to Y1, for example 5. Within the long-term criterion it may for example be checked whether 5 out of 8 cardiac cycles immediately prior to the current cardiac cycle have been missed.

Based on the evaluation of the reduction criterion the sense threshold is reduced. If it is found that the reduction criterion is fulfilled, the sense threshold is reduced in a predetermined manner. If the reduction criterion is not fulfilled, the sense threshold is not reduced based on the reduction criterion, but may be adapted in another pre-scribed manner, for example based on a peak amplitude determined for an atrial event. By reducing the sense threshold it can be made sure that atrial events for subsequent cardiac cycles can be detected with an increased reliability, as the sense threshold previously may have had a too large value, causing a significant amount of missed atrial events in prior cardiac cycles. By dynamically adjusting the sense threshold on a cycle-by-cycle basis, hence, atrial detection may be improved and a loss of atrial events may be limited.

The sensor arrangement in particular may be formed by an electrode arrangement of one or multiple electrodes arranged on the body. Hence, by means of the sensor arrangement electrical signals may be received, such electrical signals representing intracardiac electrogram recordings and hence being indicative of cardiac activity.

In another embodiment, the sensor arrangement may be configured for sensing cardiac signals in the shape of pressure signals, acoustic signals, ultrasound signals, motion signals, and/or impedance signals.

In one embodiment, the body of the implantable medical device may be formed by a lead which is connectable to a generator of the implantable medical device. In this case, the generator may be implanted into a patient for example subcutaneously remote from the heart, the lead forming the body extending from the generator into the heart such that the body with the sensor arrangement arranged thereon is placed in the heart, for example within the right ventricle in order to engage with tissue at the right ventricle.

In another embodiment, the body may be formed by a housing of a leadless pacemaker device. In this case, the implantable medical device is formed as a leadless device, which does not comprise leads extending from a location outside of the heart into the heart for providing for a stimulation and/or sensing within the heart. The housing of the leadless pacemaker device may be placed on tissue with a distal end formed by the housing, the sensor arrangement e.g. being placed (at least in part) on or in the vicinity of the distal end and engaging with tissue when placing the leadless pacemaker device on tissue with its distal end.

If the implantable medical device is a leadless pacemaker device, the housing provides for an encapsulation of the implantable medical device, the implantable medical device including all required components for autonomous operation, such as the processing circuitry, an energy storage such as a battery, electric and electronic circuitry and the like, within the housing. The housing is fluid-tight such that the implantable medical device may be implanted into cardiac tissue and may be kept in cardiac tissue over an extended period of time to provide for a long-term, continuous cardiac pacing operation.

According to the invention, the processing circuitry is configured to reduce the sense threshold for a detection of an atrial event in a current cycle if in the current cycle it is found that said reduction criterion is fulfilled, wherein the reduction criterion is fulfilled if in X1 out of Y1 cardiac cycles prior to the current cycle no atrial events have been detected. Hence, in a current cycle it is checked whether the reduction criterion is fulfilled for cardiac cycles (immediately) prior to the current cycle. Herein, Y1 cardiac cycles prior to the current cycle are examined, and it is checked whether in X1 out of those Y1 cardiac cycles no atrial events have been detected. If this is the case, the reduction criterion is fulfilled and the sense threshold is suitably reduced. If not, the reduction criterion is not fulfilled and the sense threshold is not reduced according to the prescribed manner associated with the reduction criterion.

In one embodiment, the processing circuitry is configured to reduce the sense threshold by a reduction factor if said reduction criterion is fulfilled. The reduction factor may be a percentage value which is applied to compute the sense threshold if the reduction criterion is fulfilled. The reduction factor may be programmable, for example in steps in between 5 to 10%, for example 6.25%, and may assume a value between 0 and 100%.

The reduction factor may be applied to for example an average threshold reference value, based on which the sense threshold is generally set. Alternatively, the reduction factor may be applied to the previously valid sense threshold.

In one embodiment, in addition to reducing the sense threshold by the predefined reduction factor an additional step factor may be applied if the reduction criterion is fulfilled for two or more consecutive cycles. Generally, the reduction factor is applied if for the current cycle the reduction criterion is fulfilled. If for at least one subsequent cardiac cycle the reduction criterion is again fulfilled, an additional step factor may be applied. If the reduction criterion is again fulfilled for the next cardiac cycle, the step factor may be applied again, such that the step factor may be applied repeatedly if for multiple cardiac cycles the reduction criterion is fulfilled.

The step factor may also assume a percentage value and may also be programmable, for example in steps in between 5 to 10%, for example 6.25%, and may assume a value between 0 and 100%.

The additional step factor may be applied for example to the previously valid sense threshold, in particular to the sense threshold after application of the reduction factor.

In one embodiment, multiple reduction criteria may be applied. For example, in addition to the reduction criterion described above another, second reduction criterion may be applied, for which it is evaluated whether in X2 out of Y2 cardiac cycles no atrial events have been detected. X2 herein is a natural number larger than X1, and Y2 is a natural number larger than Y1.

As X2 and Y2 are larger than X1 and Y2, the second reduction criterion is a long-term criterion to check whether over a prolonged period a significant number of atrial events in cardiac cycles have been missed. The first reduction criterion, for which it is evaluated whether in X1 cardiac cycles out of Y1 cardiac cycles atrial events have been missed, in this case may be a short-term criterion, for which in a small number of recent cardiac cycles it is evaluated whether a significant number of atrial events have been missed.

If the second reduction criterion is fulfilled, the processing circuitry may be configured, in one embodiment, to reduce the sense threshold by a second reduction factor associated with the second reduction criterion. The second reduction factor generally is larger than the first reduction factor associated with the first reduction criterion and may be programmable, for example in steps between 5 to 10%, for example 6.25%, and may have a value between 0 to 100%.

The second reduction factor may be applied to for example an average threshold reference value, based on which the sense threshold is generally set. Alternatively, the second reduction factor may be applied to the previously valid sense threshold.

In one embodiment, if both the first reduction criterion and the second reduction criterion are fulfilled, only the second reduction factor is applied. The second reduction criterion hence takes precedence over the first reduction criterion, such that in case that both reduction criteria are fulfilled the sense threshold is reduced by means of the second reduction factor.

In one embodiment, in addition to reducing the sense threshold by the predefined second reduction factor an additional second step factor may be applied if the second reduction criterion is fulfilled for two or more consecutive cycles. Generally, the second reduction factor is applied if for the current cycle the second reduction criterion is fulfilled. If for the subsequent cardiac cycle or multiple cardiac cycles the second reduction criterion is again fulfilled, the additional second step factor may be applied. If the second reduction criterion is again fulfilled for the next cardiac cycle, the second step factor may be applied again, such that the second step factor may be applied repeatedly if for multiple cardiac cycles the second reduction criterion is fulfilled.

The second step factor may also assume a percentage value and may also be programmable, for example in steps between 5 to 10%, for example 6.25%, and may assume a value between 0 and 100%.

The additional step factor may be applied for example to the previously applied setting of the sense threshold, in particular to the sense threshold after application of the second reduction factor.

In one embodiment, the processing circuitry is configured not to reduce the sense threshold beyond a lower absolute threshold. Hence, the sense threshold is limited to a lower bound, defined by the lower absolute threshold. Even if the first reduction criterion and/or the second reduction criterion is (repeatedly) fulfilled while the sense threshold is at the lower bound, no further reduction of the sense threshold takes place.

In one embodiment, the processing circuitry is configured to determine a peak amplitude associated with a detected, valid atrial event. If an atrial event is detected, data recorded for the atrial event is analyzed in order to determine the peak amplitude. For example, the atrial event is identified as that point in time at which the cardiac sense signal crosses a sense threshold, also denoted as atrial detection threshold. Beginning with the crossing of the sense threshold a peak detection window is started, and within the peak detection window the largest sense signal value is searched for, which then is assumed as the peak amplitude.

A crossing of the sense threshold may be found if one value of a (processed) cardiac sense signal is larger than the atrial detection threshold. As far-field atrial signals may be noisy, in another embodiment it may be assumed that an atrial event is present if and only if two or more samples are larger than the sense threshold, wherein these examples may be consecutive or may be not consecutive.

Using the peak amplitude, the processing circuitry may be configured to update the sense threshold for detecting a subsequent atrial event. In particular, the processing circuitry may be configured to update the sense threshold using an average threshold reference and a percentage ratio according to the formula

$$ST = PC \cdot ATR(t),$$

where ST is the current sense threshold, PC is a percentage ratio, and ATR(t) is the current average threshold reference for cycle t. The percentage ratio may lie for example in the range between 0 % and 100 % and may be programmable.

The average threshold reference may be computed and updated based on the peak amplitude according to the following equation:

$$ATR(t) = W \cdot PA(t\text{-}1) + (1\text{-}W) \cdot ATR(t\text{-}1),$$

where W indicates the update weight which determines how much the average threshold reference should change based on the previous peak amplitude..., PA(t-1) is the peak amplitude as determined for the previous cycle t-1, and ATR(t-1) is the previous average threshold reference.

For the actual cycle t the average threshold reference hence is determined based on the peak amplitude determined for that cycle and based on the previously valid average threshold reference at cycle t-1. For each cycle, hence, the average threshold reference is updated and computed anew, such that the average threshold reference is dynamically adjusted on a cycle-by-cycle basis.

In another embodiment, the average threshold reference may be determined as a mean value of peak amplitudes for a predefined number of cardiac cycles in which atrial events have been detected, for example a number in between two to six cardiac cycles, for example four cardiac cycles.

The peak amplitude is determined only if a (valid) atrial event is detected. If no atrial event is detected, the peak amplitude is not determined, and the average threshold reference is not updated. In this case, the cycle is counted as a missed cycle, i.e. a cycle in which no atrial activity was sensed.

In one embodiment, the processing circuitry comprises a first processing channel having a first gain for processing a first processing signal derived from signals received via the sensor arrangement and a second processing channel having a second gain for processing a second processing signal derived from signals received via the sensor arrangement, the second gain being higher than the first gain.

Generally, the implantable medical device may be configured to process different processing signals. For obtaining such processing signals, a sensor arrangement is provided, the sensor arrangement comprising e.g. one or multiple electrodes to receive electrical signals from which the processing signals are derived. The processing signals herein, for example, may be obtained each using a pair of electrodes, wherein for obtaining the different processing signals the same pair of electrodes or different pairs of electrodes may be used. **In** the first case, a single electrical signal, such as an intracardiac electrogram, may be obtained, from which different processing signals, namely the first processing signal and the second processing signal are derived for separate processing. In the latter case, separate electrical signals relating for example to a ventricular sensing signal and an atrial sensing signal (i.e., by applying a sensing optimized for atrial sensing) may be received in order to derive the first processing signal and the second processing signal from such different electrical signals, the different electrical signals for example being received using different pairs of electrodes of the sensor arrangement.

The different processing signals, in one embodiment, are processed in different processing paths of the processing circuitry. For this, the processing circuitry comprises a first processing channel for processing the first processing signal, the first processing signal relating for example to a near-field (in particular ventricular) sensing signal which, according to the placement of the implantable medical device for example in a ventricle of a patient's heart, may be large such that the first processing channel may exhibit a rather low gain.

In addition, the processing circuitry comprises a second processing channel for processing the second processing signal, which may relate for example to a far-field atrial sensing signal which, in case of a placement of the implantable medical device in the ventricle, may have a small amplitude, due to the distance between the location of implantation and the source of origin of the signals. In order to allow for a reliable processing of the second processing signal, the second processing channel exhibits a gain higher than the gain of the first processing channel, such that features relating to atrial activity may be suitably analyzed within the received signals.

Because, for a placement of the implantable medical device in for example the ventricle, atrial activity occurs in the far field, atrial events within a regular ventricular sensing signal (for example obtained from a regular ventricular QRS sensing channel) may be hard to discern, as a P wave stemming from atrial activity may exhibit a small amplitude in relation to QRS and T waves. For this reason signal portions relating to far-field activity may be processed separately from signals relating to near-field activity within the second processing channel, such that within the second processing channel far-field events may be detected with increased reliability and enhanced timing precision.

The implantable medical device, in one aspect, is to be placed entirely or partially in the right or left ventricle.

In one aspect, the sensor arrangement is formed by an electrode arrangement, the electrode arrangement comprising a first electrode arranged in the vicinity of a tip of the body. The first electrode shall come to rest on cardiac tissue in an implanted state of the implantable medical device, such that the first electrode contacts cardiac tissue e.g. at a location effective for injecting a stimulating signal into cardiac tissue for provoking a pacing action, in particular ventricular pacing.

In one aspect, the electrode arrangement comprises a second electrode formed by an electrode ring circumferentially extending about the body. Alternatively, the second electrode may for example be formed by a patch or another electrically conductive area formed on the body. The second electrode is placed at a distance from the tip of the body and hence at a distance from the first electrode arranged at the tip.

In one embodiment, the processing circuitry is configured to process, as said first processing signal, a first signal sensed between the first electrode and the second electrode. Such first signal may be denoted as near-field vector to be received between a pair of electrodes comprised of the first electrode and the second electrode. As the first electrode and the second electrode may, in one embodiment, be located at a rather close distance to each other, such pair of electrodes is predominantly suited to receive signals in close proximity to the implantable medical device, i.e., in the near-field region within the ventricle if the implantable medical device is implanted into the ventricle. The sense signal received in between the first electrode and the second electrode is provided to the first processing channel for processing in order to for example detect near-field (e.g., ventricular) events in the signal.

In one embodiment, the body comprises a remote location (e.g. the far end of a housing of a leadless pacemaker device) removed from the tip, the electrode arrangement comprising a third electrode arranged on the body at the remote location. The third electrode is operatively connected to the processing circuitry, such that the processing circuitry is enabled to receive and process signals received via the third electrode.

In one aspect, the processing circuitry is configured to process, as said second processing signal, a second signal sensed between the first electrode and the third electrode. Such second signal vector arising between the first electrode and the third electrode may be referred to as far-field vector, the first electrode and the third electrode exhibiting a distance with respect to each other larger than the first and the second electrode. The second signal may in particular be processed to detect events in the far-field, i.e., atrial contractions in case the implantable medical device is placed in the ventricle, such that by means of the second signal an intrinsic atrial activity prior to injecting a pacing stimulus may be captured.

The second signal sensed between the first electrode and the third electrode may be used to sense intrinsic atrial contractions in order to provide for an atrial-to-ventricular synchronization by timely injecting a stimulus at the ventricular location of implantation of the pacemaker device following atrial contractions. The second signal is provided to the second processing channel in order to process the signal and detect atrial events from the signal, in order to provide for a pacing action based on detected atrial events, hence allowing for a ventricular pacing under atrioventricular (AV) synchrony.

In one embodiment, the second processing channel comprises a processing stage for differentiating one wave portion from another wave portion in the second processing signal. The processing stage in particular may be configured to apply, to the second processing signal, at least one of a bandpass filtering, a blanking window for excluding a portion of the second signal from further processing, a moving average filtering, and a rectification. By means of the processing stage, in particular such wave portions shall be isolated and/or emphasized within the signal to be processed which may be indicative of e.g. an atrial event. If the implantable medical device is placed in the ventricle of a patient's heart, signal portions relating to far-field atrial activity may have a much smaller amplitude than signal portions relating to a near-field ventricular activity. Hence, the processing serves to differentiate between the different signal portions in order to identify such signal portion which may contain signals relating to far-field atrial activity.

For isolating e.g. the P wave in an intracardiac electrogram, a bandpass filtering may be applied, hence differentiating wave portions relating to the P wave from wave portions in particular relating to QRS and T waves stemming from ventricular activity. Alternatively or in addition, a blanking method may be applied in order to blank out certain portions of the second processing signal, namely such portions which contain signals stemming from events other than a far-field atrial activity. A blanking window, for this, serves to silence signal portions which are not of interest for far-field activity, but which may rather interfere with the detection of far-field activity. By means of a blanking window such portions of the signal which do not relate to far-field atrial activity hence are excluded from processing, such that the processing is limited to those signal portions (likely) relating to far-field activity. Alternatively or in addition, other methods such as a moving average filtering, finite differences or a rectification of the signal may be applied. A moving averaging filter herein can be used to smooth the processing signal. Rectification can serve to easily compare the processed signal to a (single) threshold in order to identify when the signal magnitude exceeds a predefined threshold.

In one embodiment, the first processing channel comprises a first detection stage for detecting at least one near-field event in the first signal. Herein, the processing stage of the second processing channel may be configured to determine at least one limit of a blanking window for excluding a portion of the second signal from further processing based on a near-field (e.g. ventricular) event detected by the first detection stage of the first processing channel. The first processing channel serves to process a processing signal at a lower gain for detecting near-field events, i.e., events that are due to an activity in close proximity to the implanted implantable medical device, for example in the ventricle in which the implantable medical device is implanted. As the near-field event will also be picked up in the second processing signal, it is advantageous to blank out such a signal portions relating to a near-field activity, i.e., QRS and T waves in in case of a placement of the implantable medical device in the ventricle. In order to correctly locate the blanking window, detected near-field events may be taken into account, in order to for example determine a regular timing in between atrial events and ventricular events. From detected near-field events it may be determined in what time range after a far-field event a near-field event typically should occur, such that the blanking window, defined by a start time and a stop at time, may be appropriately set to blank out such portions of the second processing signal which relates to the near-field ventricular events.

During the blanking window the second processing channel may, at least partially, be switched off in order to for example safe power within the second, high gain processing channel. The second processing channel may for example comprise an amplification stage for amplifying the second processing signal, wherein the amplification stage may be switched off during the period of the blanking window such that that no power is consumed by amplification during the time interval of the blanking window.

A detection of far-field atrial events takes place in a detection window outside a blanking window. The detection window herein may start (immediately) at the end of a prior blanking window and may end at the beginning of the next blanking window. It however is also conceivable that the detection window for example starts at some time delay after the end of a prior blanking window. In between the end of the blanking window and the start of the detection window the second processing channel may be fully functional and process the associated second processing signal, wherein however a detection of far-field (atrial) events does not take place until the start of the detection window.

In one embodiment, the second processing channel comprises a second detection stage for detecting an atrial event in the second processing signal. The second detection stage may be arranged logically behind the processing stage of the second processing channel, such that the second detection stage receives processed signals from the processing stage of the second processing channel. The second detection stage herein serves to identify far-field atrial events in the second processing signal in order to output information relating to the timing of a detected atrial event.

The second detection stage of the second processing channel in particular may be configured to detect an atrial event by comparing the second processing signal to a sense threshold. In case the magnitude of the second processing signal exceeds the sense threshold, it may be concluded that a far-field atrial event is present. The processing herein may take place on a rectified signal, which makes it possible to apply a single threshold to which the rectified signal may be compared. It however is also possible to identify a far-field atrial event in a non-rectified signal, for example by applying two thresholds, namely a positive threshold and a negative threshold, wherein a far-field atrial event is identified if the positive signal portion exceeds the positive threshold and/or the (magnitude of the) negative signal portion exceeds the negative threshold.

The advantages and advantageous embodiment described above for the device equally apply also to the method, such that is shall be referred to the above.

The various features and advantages of the present invention may be more readily understood with reference to the following detailed description and the embodiments shown in the drawings. Herein,

Fig. 1 shows a schematic view of the human heart, with an implantable medical device in the shape of a leadless pacemaker device implanted therein;

Fig. 2 shows a schematic view of an implantable medical device;

Fig. 3 shows a schematic view of an implantable medical device, indicating signal vectors between different electrodes of the implantable medical device;

Fig. 4 shows a schematic view of a processing circuitry of an embodiment of an implantable medical device;

Fig. 5A shows a first processing signal in the shape of an intracardiac electrogram (IEGM) processed by a first processing channel of the processing circuitry;

Fig. 5B shows a second processing signal processed by a second processing channel of the processing circuitry;

Fig. 6 shows an example of a sense threshold adapted over a multiplicity of cardiac cycles based on a peak amplitude and reduction criteria;

Fig. 7 shows a schematic view of multiple cardiac cycles and an evaluation of one reduction criterion;

Fig. 8 shows an example, in a schematic view, of multiple cardiac cycles and an evaluation of two reduction criteria; and

Fig. 9     shows a schematic view of the human heart, with an implantable medical device in the shape of cardiac stimulation device having a lead implanted in the right ventricle.

Subsequently, embodiments of the invention shall be described in detail with reference to the drawings. In the drawings, like reference numerals designate like structural elements.

It is to be noted that the embodiments are not limiting for the invention, but merely represent illustrative examples. The scope of the present invention is defined by the appended claims.

In the instant invention it is proposed to provide an implantable medical device providing for an intracardiac function, in particular a ventricular pacing, specifically a so-called VDD pacing.

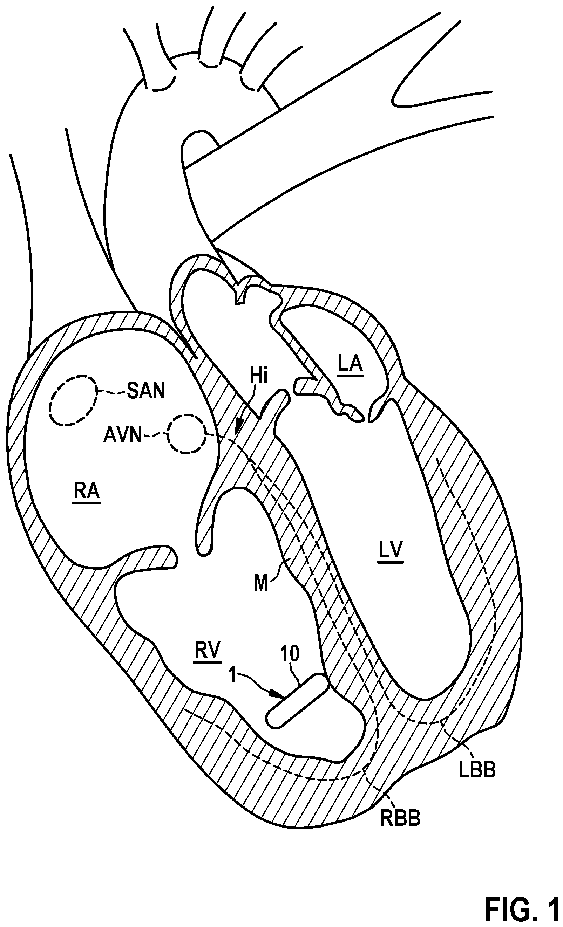

Fig. 1 shows, in a schematic drawing, the human heart comprising the right atrium RA, the right ventricle RV, the left atrium LA and the left ventricle LV, the so-called sinoatrial node SAN being located in the wall of the right atrium RA, the sinoatrial node SAN being formed by a group of cells having the ability to spontaneously produce an electrical impulse that travels through the heart's electrical conduction system, thus causing the heart to contract in order to pump blood through the heart. The atrioventricular node AVN serves to coordinate electrical conduction in between the atria and the ventricles and is located at the lower back section of the intra-atrial septum near the opening of the coronary sinus. From the atrioventricular node AVN the so-called HIS bundle H is extending, the HIS bundle H being comprised of heart muscle cells specialized for electrical conduction and forming part of the electrical conduction system for transmitting electrical impulses from the atrioventricular node AVN via the so-called right bundle branch RBB around the right ventricle RV and via the left bundle branch LBB around the left ventricle LV.

In case of a block at the atrioventricular node AVN, the intrinsic electrical conduction system of the heart H may be disrupted, causing a potentially insufficient intrinsic stimulation of ventricular activity, i.e., insufficient or irregular contractions of the right and/or left ventricle RV, LV. In such a case, a pacing of ventricular activity by means of a pacemaker device may be indicated, such pacemaker device stimulating ventricular activity by injecting stimulation energy into intracardiac tissue, specifically myocardium M.

In one embodiment, an implantable medical device 1 in the shape of a leadless cardiac pacemaker device, as schematically indicated in Fig. 1, is provided for a ventricular pacing action, the leadless pacemaker device having a body 10 formed by the housing of the leadless pacemaker device.

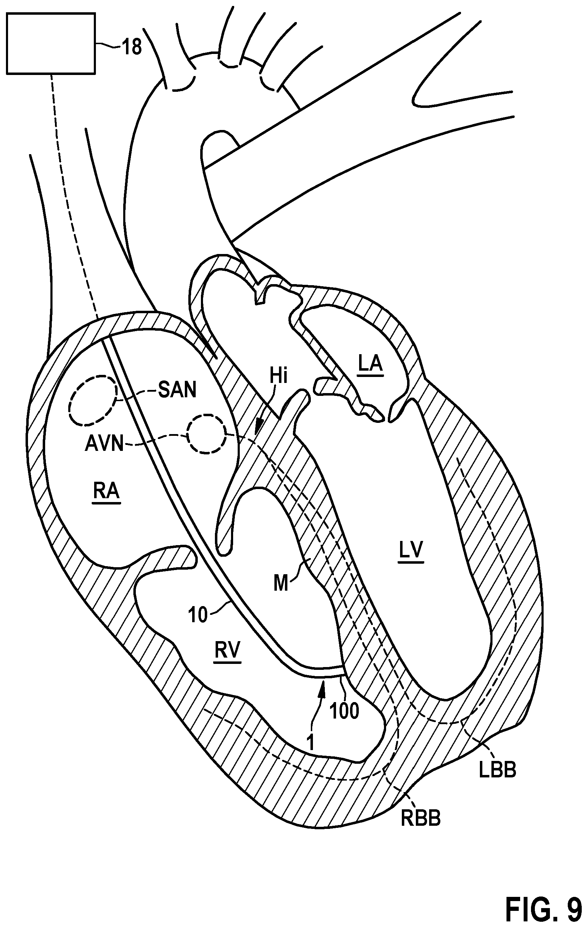

In another embodiment, as shown in Fig. 9, the implantable medical device 1 may be a stimulation device having a generator 18 and at least one lead forming a body 10 of the implantable medical device 1 and extending transvenously from the generator 18 into the patient's heart.

Whereas common implantable medical devices are designed to sense a ventricular activity by receiving electrical signals from the ventricle RV, LV they are placed in, it may be desirable to provide for a pacing action which achieves atrioventricular (AV) synchrony by providing a pacing in the ventricle in synchrony with an intrinsic atrial activity. For such pacing mode, also denoted as VDD pacing mode, it is required to sense atrial activity and identify atrial events relating to atrial contractions in order to base a ventricular pacing on such atrial events.

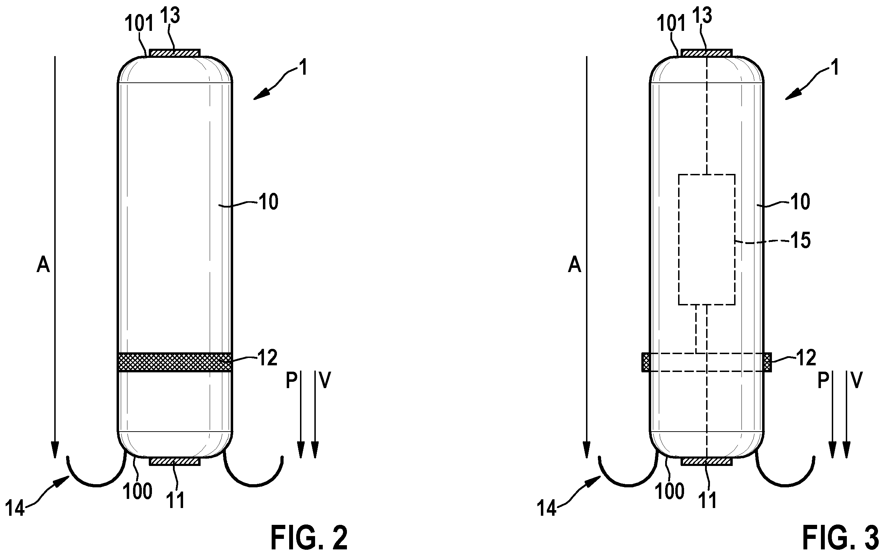

Referring now to Figs. 2 and 3, in one embodiment an implantable medical device 1 in the shape of a leadless pacemaker device configured to provide for an intracardiac pacing, in particular in a VDD pacing mode, comprises a housing 10 enclosing electrical and electronic components for operating the implantable medical device 1. In particular, enclosed within the housing 10 is a processing circuitry 15, comprising for example also a communication interface for communicating with an external device, such as a programmer wand. In addition, electrical and electronic components such as an energy storage in the shape of a battery are confined in the housing 10. The housing 10 provides for an encapsulation of components received therein, the housing 10 having the shape of, e.g., a cylindrical shaft having a length of for example a few centimeters.

The implantable medical device 1 is to be implanted immediately on intracardiac tissue M. For this, the implantable medical device 1 comprises, in the region of a tip 100, a fixation device 14 for example in the shape of nitinol wires to engage with intracardiac tissue M for fixedly holding the implantable medical device 1 on the tissue in an implanted state.

The implantable medical device 1 in the embodiment of Figs. 2 and 3 does not comprise leads, but receives signals relating to a cardiac activity by means of an electrode arrangement arranged on the housing 10 and also emits stimulation signals by means of such electrode arrangement. In the embodiment of Figs. 2 and 3, the implantable medical device 1 comprises different electrodes 11, 12, 13 making up the electrode arrangement and serving to emit pacing signals towards intracardiac tissue M for providing a pacing and to sense electrical signals indicative of a cardiac activity, in particular indicative of atrial and ventricular contractions.

A first electrode 11 herein is denoted as pacing electrode. The first electrode 11 is placed at a tip 100 of the housing 10 and is configured to engage with cardiac tissue M.

A second electrode 12 herein is denoted as pacing ring. The second electrode 12 serves as a counter-electrode for the first electrode 11, a signal vector P arising between the first electrode 11 and the second electrode 12 providing for a pacing vector P for emitting pacing signals towards the intracardiac tissue M.

In addition, the second electrode 12 serves as a sensing electrode for sensing signals, in particular relating to ventricular contractions, a signal vector V arising between the second electrode 12 and the first electrode 11, the signal vector V being denoted as near-field vector.

The second electrode 12 is placed at a distance from the first electrode 11 and for example has the shape of a ring extending circumferentially about the housing 10. The second electrode 12 is for example placed at a distance of about 1 cm from the tip 100 of the housing 10 at which the first electrode 11 is placed.

The implantable medical device 1, in the embodiment of Figs. 2 and 3, in addition comprises a third electrode 13 placed at a far end 101 of the housing 10, the third electrode 13 serving as a sensing electrode for sensing signals indicative of cardiac activity in the far-field. In particular, a signal vector A arises between the third electrode 13 and the first electrode 11, the signal vector A picking up signals being indicative for example of atrial contractions and being denoted as far-field vector.

The electrodes 11, 12, 13 are in operative connection with the processing circuitry 15, the processing circuitry 15 being configured to cause the first electrode 11 and the second electrode 12 to emit a pacing signal for providing a stimulation at the ventricle. The processing circuitry 15 furthermore is configured to process signals received via the electrodes 11, 12, 13 to provide for a sensing of cardiac activity, in particular atrial and ventricular contractions.

If the implantable medical device 1 has the shape of a stimulation device comprising a generator 18 and a lead extending from the generator 18, as shown in the embodiment of Fig. 9, a similar electrode arrangement comprising for example three electrodes 11, 12, 13 may be arranged on a lead implanted in and extending into the right ventricle RV, as shown in Fig. 9, such that the above also applies to an embodiment of the implantable medical device 1 having a lead extending into the patient's heart. In this case, the processing circuitry 15 may be part of the generator 18 and may be in operative connection with an electrode arrangement arranged on the lead.

In order to provide for a pacing in the ventricle in which the implantable medical device 1 is placed, in particular to enable a pacing in the VDD mode, a sensing of atrial activity is required to provide for detected atrial sense markers in order to time a pacing in the ventricle to obtain atrioventricular (AV) synchrony. For this, a far-field signal from in particular the right atrium RA (see Figs. 1 and 9) shall be sensed in order to allow for a synchronous pacing in the right ventricle RV by means of the implantable medical device 1 being implanted on intracardiac tissue M in the right ventricle RV.

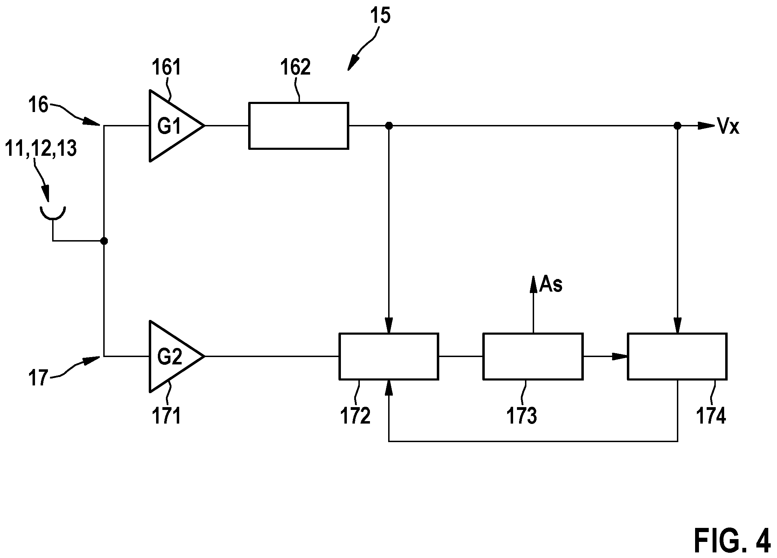

Referring now to Fig. 4, the processing circuitry 15 comprises, in one embodiment, two processing channels 16, 17 for processing different processing signals relating to ventricular activity and atrial activity. Herein, typically, an intracardiac electrogram (IEGM) contains a signal portions relating to ventricular activity (in particular a QRS wave) and atrial activity (in particular a P wave), signal portions relating to atrial activity however resulting from a far-field signal source and hence being far less pronounced and having a far smaller amplitude then signal portions relating to a ventricular activity in the near-field, i.e., arising in close proximity to the implanted implantable medical device 1. For this reason, the two processing channels 16, 17 are associated with different gains G1, G2, a first processing channel 16 serving to process a first processing signal to identify ventricular events at a rather low gain G1 and a second processing channel 17 being configured to process a second processing signal to identify atrial events at a significantly higher gain G2.

In particular, the first processing channel 16 is connected to the electrode arrangement comprised of the electrodes 11, 12, 13, the first processing channel 16 being configured in particular to sense and process a signal received via the electrodes 11, 12 (near-field vector V in Figs. 2 and 3). The first processing channel 16 comprises a first amplification stage 161 having a gain G1 and, following the amplification stage 161, a detection stage 162 which is configured to identify ventricular sense markers Vx from the first processing signal processed within the first processing channel 16.

The second processing channel 17 is likewise connected to the electrode arrangement comprised of electrodes 11, 12, 13, wherein the second processing channel 17 may in particular be configured to process a signal sensed via the far-field vector A, that is in between the electrodes 11, 13 placed at the tip 100 and the far end 101 of the housing 10 as illustrated in Figs. 2 and 3. The second processing channel 17 comprises a second amplification stage 171 having a second gain G2, the second amplification stage 171 being followed by a processing stage 172 and a second detection stage 173.

The processing stage 172 serves to pre-process the second processing signal after amplification. The detection stage 173 in turn serves to evaluate and analyze the processed signal in order to identify atrial events within the second processing signal, the second processing channel 17 then outputting atrial sense markers As indicative of atrial events detected in the processed signal.

In addition, the processing circuitry 15 comprises a timing stage 174 which uses timing information received from the first processing channel 16 and the second processing channel 17 to provide for a pacing timing, in particular a VDD timing for achieving an atrial-ventricular synchronous pacing.

In order to identify and analyze atrial events, the gain G2 of the second processing channel 17 is (significantly) higher than the gain G1 of the first processing channel 16. This generally allows to analyze signal portions relating to atrial events, but makes it necessary to discern such signal portions relating to atrial events from other signal portions, in particular signal portions relating to ventricular events in the near-field and hence being far stronger than signal portions originating from atrial events in the far-field.

Within the processing stage 172, for example a bandpass filtering, a windowing (e.g. partial blanking), a smoothing by means of a moving average filtering and a rectification may take place. A first or second order difference may be applied to remove a non-zero baseline while enhancing P wave defections.

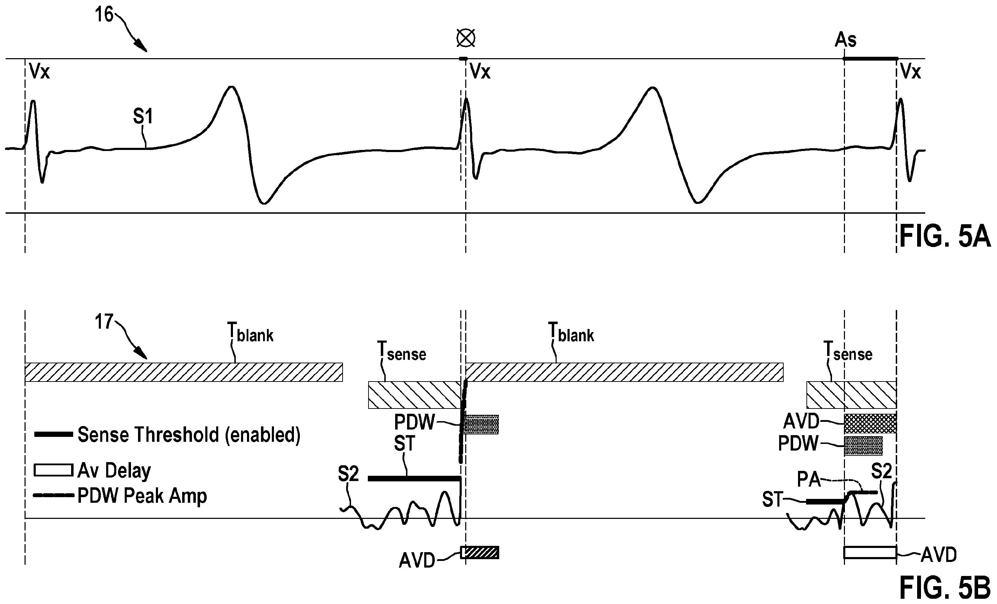

Figs. 5A and 5B show examples of signals S1, S2 as processed in the different processing channels 16, 17, Fig. 5A at the top showing a signal S1 as processed by the first processing channel 16 and Fig. 5B at the bottom showing a signal S2 as processed by the second processing channel 17. As a result of the processing, ventricular events Vx and atrial events As are identified and corresponding markers are output.

As apparent from Fig. 5B, the sensing of atrial events As uses a windowing scheme, employing in particular a blanking window $T_{blank}$ for blanking out signal portions of the signal S2 potentially relating to ventricular activity.

In particular, by means of the detection of ventricular events Vx in the first processing channel 16 a timing in between atrial events As and ventricular events Vx may be determined. According to such timing a start point and an end point of the blanking window $T_{blank}$ may be set, hence excluding signal portions from the processing which do not relate to atrial activity. Strong ventricular signals in this way may be suppressed such that signal portions relating to a ventricular activity may not interfere with a detection of atrial events.

During the blanking window $T_{blank}$, the second processing channel 17 may be turned off. In particular, the amplification stage 171 of the second processing channel 17 may be switched of in order to save power.

Generally, a detection for atrial events takes place outside of the blanking window $T_{blank}$. Herein, a detection window $T_{sense}$ for detecting atrial events may start at the end of a prior blanking window $T_{blank}$. Alternatively, a detection window $T_{sense}$ may - as shown in the embodiment of Fig. 5B - have a delay with respect to the end of a prior blanking window $T_{blank}$, such that a signal processing within the second processing channel 17 starts at the end of a prior blanking window $T_{blank}$, a detection for atrial events however starting only after a certain delay.

Generally, an atrial event As is assumed to be present if, in the sense window $T_{sense}$, the signal S2 crosses a sense threshold ST, as it is shown in Fig. 5B. The comparison may take place based on a rectification of the sense signal S2. Alternatively, a positive and negative sense threshold ST may be used, which may have the same value or may differ in their values. A threshold crossing herein may be assumed if one signal value is larger than the sense threshold ST. Alternatively, a crossing of the sense threshold ST is assumed if a predefined number of signal values are larger than the sense threshold ST, for example two or more consecutive sample values.

Generally, if an atrial event As is detected, as it is the case for the second cardiac cycle in Fig. 5B, the atrial event As is used for a further processing, in particular to update the sense threshold ST and to achieve an atrial-ventricular synchronous pacing.

In particular, the atrial event As is taken as that point in time at which a crossing of the sense threshold ST is identified. At the time of the atrial event As a peak detection window PDW starts, and based on data recorded during that peak detection window PDW a peak amplitude PA is determined as the maximum signal value within the peak detection window PDW. This is indicated in Fig. 5B for the second cycle at the right.

Also, in case of a detection of an atrial event As, an atrial-ventricular delay AVD may be determined and used for subsequent processing. If no ventricular event Vx is detected after lapse of the atrial-ventricular delay AVD, a pacing signal may be injected to cause a ventricular stimulation.

The peak amplitude PA, in one embodiment, may be used to update the sense threshold ST. In particular, the processing circuitry 15 may be configured to update the sense threshold ST using an average threshold reference and a percentage ratio according to the formula

$$ST = PC \cdot ATR(t),$$

where ST is the current sense threshold, PC is the percentage ratio, and ATR(t) is the average threshold reference for the current cycle t. The percentage ratio may lie for example in the range between 0 % and 100 %.

The average threshold reference may be determined based on a mean value for a number of previous cardiac cycles in which atrial events have been identified and correspondingly peak amplitude values have been obtained. The average threshold reference in this case for example may be determined as the average of the peak amplitude values in the previous cardiac cycles.

In another embodiment, the average threshold reference may be computed based on the peak amplitude PA according to the following equation:

$$ATR(t) = W \cdot PA(t\text{-}1) + (1\text{-}W) \cdot ATR(t\text{-}1),$$

where W indicates the update weight which determines how much the average threshold reference should change based on the previous peak amplitude..., PA(t-1) is the peak amplitude as determined for the previous cycle t-1, and ATR(t-1) is the previous average threshold reference.

For the actual cycle t the average threshold reference hence is determined based on the peak amplitude PA determined for that cycle t and based on the previously valid average threshold reference at cycle t-1. For each cycle for which an atrial event As is detected, hence, the average threshold reference is updated and computed anew, such that the average threshold reference is dynamically adjusted on a cycle-by-cycle basis.

If no (valid) atrial event As is detected, no peak amplitude PA is determined and the average threshold reference ATR is not updated. In this way it is avoided that a false detection of an atrial event As may cause a false increase of the sense threshold ST and a subsequent capture loss of atrial activity. This is the case for the first cardiac cycle as shown in Figs. 5A and 5B, in which no crossing of the sense threshold ST is detected and correspondingly no atrial event As is identified.

The sense threshold ST generally is set based on the peak amplitude value PA of detected atrial events As in previous cardiac cycles. Correspondingly, dependent on the peak amplitude values in the previous cardiac cycles the sense threshold ST may be dynamically raised or lowered.

This however only applies if atrial events As are detected. If no atrial event As is detected in a cardiac cycle, no peak amplitude PA is determined, and no dynamic adjustment of the sense threshold based on the above-noted scheme takes place. However, as there is a general desire to reliably detect atrial events As and to obtain a steady, reliable capture of atrial events As to obtain atrial events As in as many cycles as possible, in case of a missing of a significant number of atrial signals measures should be taken to recapture signals associated with atrial activity. A missing of one or multiple atrial events As in cardiac cycles may indicate that the sense threshold ST is too high and hence should be lowered in order to recapture atrial activity.

For this reason it is proposed to dynamically adjust the sense threshold ST in a step decay procedure if, based on one or multiple reduction criteria, it is found that a significant number of atrial events As are missed for cardiac cycles. These reduction criteria are formulated as "X out of Y criteria". A reduction criterion is fulfilled, correspondingly, if in X out of Y cardiac cycles no atrial events As are detected.

The reduction criteria herein may include a short-term criterion and a long-term criterion.

In a short-term criterion, for example, it may be evaluated whether in X1 out of Y1 cardiac cycles atrial events As have not been detected. If this is the case, the short-term criterion is assumed to be true and the sense threshold ST is adjusted in a suitable manner. X1 and Y1 herein are natural numbers, where X1 is larger than then 0 and Y1 is equal to or larger than X1. Within the short-term criterion herein it shall be checked whether in very recent cardiac cycles a significant number of atrial events As are missed. Y1 may correspondingly take values for example in between 1 to 4, and X1 may assume a value between 1 and Y1.

For example, Y1 may be equal to 2, and X1 may be equal to 1 or 2. It hence is checked whether in one out of two previous cardiac cycles atrial events are missed, or whether in 2 out of 2 previous cardiac cycles atrial events are missed.

In a long-term criterion it may for example be evaluated whether in X2 out of Y2 cardiac cycles atrial events As have not been detected. If this is the case, the long-term criterion is assumed to be true and the sense threshold ST is adjusted in a suitable manner. X2 and Y2 are natural numbers, where Y2 is larger than Y1 and may for example have a value between 4 and 16, and X2 is larger than X1 and smaller or equal to Y2 and may for example have a value between 3 to 15.

For example, Y2 may be equal to 8, and X2 may be equal to 5. It hence is checked whether in 5 out of 8 previous cardiac cycles atrial events As have been missed.

If the first reduction criterion, corresponding to the short-term reduction criterion, is fulfilled, a first reduction factor may be applied. If alternatively or in addition the second reduction criterion is fulfilled, corresponding to the long-term reduction criterion, a second reduction factor may be applied, wherein the second reduction factor may cause a stronger reduction than the first reduction factor.

Herein, in one embodiment, if the second reduction criterion is fulfilled only the second reduction factor is applied.

The first reduction factor and the second reduction factor may both be percentage values. The reduction factors may be programmable, for example in steps between 5 to 10%, for example 6.25%. Each reduction factor may assume a value between 0 to 100%, wherein the second reduction factor generally may cause a stronger reduction than the first reduction factor.

The respective reduction factor is applied if the respective reduction criterion is fulfilled for a current cardiac cycle. In addition, if for multiple consecutive cardiac cycles the respective reduction criterion is repeatedly fulfilled, in addition to the reduction factor an additional step factor may be applied to further reduce the sense threshold.

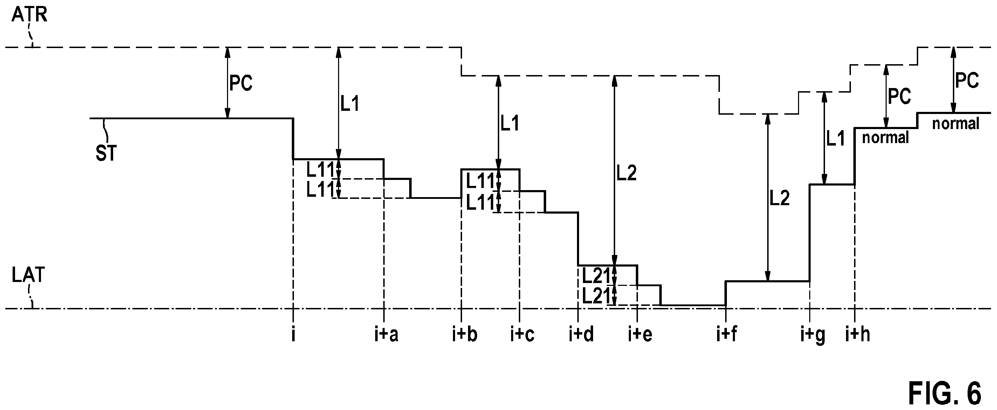

This is illustrated in Fig. 6.

Generally, if no reduction criterion is fulfilled, the sense threshold ST is computed based on the average threshold reference ATR (which is computed based on peak amplitude values PA of previous cardiac cycles) by applying a predefined, normal percentage ratio PC, the percentage ratio having for example a value in between 40% and 100%, for example 80%. The percentage ratio PC is applied as a factor, the sense threshold ST hence taking a value of PC times ATR.

If in a cardiac cycle $i$ it is found that a first reduction criterion, corresponding for example to a short-term reduction criterion, is fulfilled, the sense threshold ST is reduced by a first reduction factor L1. The first reduction factor L1 for example is applied to the valid average threshold reference ATR, such that the sense threshold ST is computed for example as L1 times the current value of the average threshold reference ATR.

If in a predefined number of multiple consecutive cycles the first reduction criterion is fulfilled, an additional step decay may be applied by multiplying the currently valid sense threshold ST with a step factor L11, wherein the step factor L11 may be repeatedly applied, as shown in Fig. 6. The step factor L11 may be applied if in two consecutive cycles the reduction criterion is fulfilled. The step factor L11 may alternatively be applied only if in more than two consecutive cycles the reduction criterion is fulfilled, as this is shown in Fig. 6 at the cardiac cycle *i+a.*

If in a cardiac cycle *i+b* an atrial event is detected and the first reduction criterion is no longer fulfilled, the average threshold reference ATR is adjusted according to the now determined peak amplitude PA, and the sense threshold ST is determined by reverting the step factors L11 and by only applying the reduction factor L1 to the average threshold reference ATR.

If at cycle *i+c* again it is found that the first reduction criterion has been fulfilled for the predefined number of consecutive cycles, the step factor L11 is again applied to reduce the sense threshold ST.

At cardiac cycle *i+d* it is found that (potentially in addition to the first reduction criterion) also the second reduction criterion is fulfilled, in which case the reduction factor L2 associated with the second reduction criterion is applied to compute the sense threshold ST. The second reduction factor L2 causes an emphasized decrease of the sense threshold ST, as apparent from Fig. 6.

At cardiac cycle *i+e* it is found that the second reduction criterion has been fulfilled for a predefined number of consecutive cardiac cycles, such that additional step factors L21 associated with the second reduction criterion are applied to further reduce the sense threshold ST.

Herein, as visible from Fig. 6, the sense threshold ST cannot be reduced beyond a lower absolute threshold LAT, which serves as a lower bound and hence represents the absolute minimum of the sense threshold ST.

At cardiac cycle *i+f* an atrial event is detected, and correspondingly the average threshold reference ATR is adjusted and the step factors L21 are reverted, such that the sense threshold ST is determined by applying only the reduction factor L2 associated with the second reduction criterion to the absolute threshold reference ATR.

At cardiac cycle *i+g* the second reduction criterion is no longer true and an atrial event is detected, and correspondingly the average threshold reference ATR is increased and the sense threshold ST is computed by applying only the reduction factor L1 associated with the first reduction criterion.

At cardiac cycle *i+h* also the first reduction criterion is no longer fulfilled and an atrial event is detected, and correspondingly the average threshold reference ATR is adjusted and the sense threshold ST is computed by applying only the percentage ratio PC indicative of normal operation.

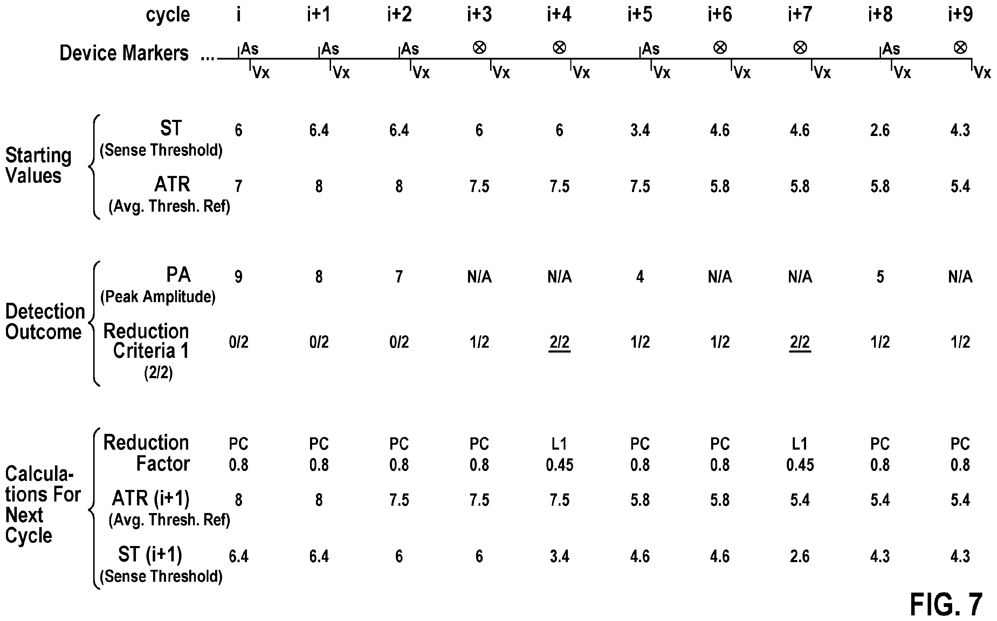

Fig. 7 shows an example of an adjustment of the sense threshold ST based on a single reduction criterion, formulated as an X out of Y criterion in which X and Y are equal to 2. It hence is evaluated whether in 2 out of 2 cardiac cycles no atrial events As have been detected.

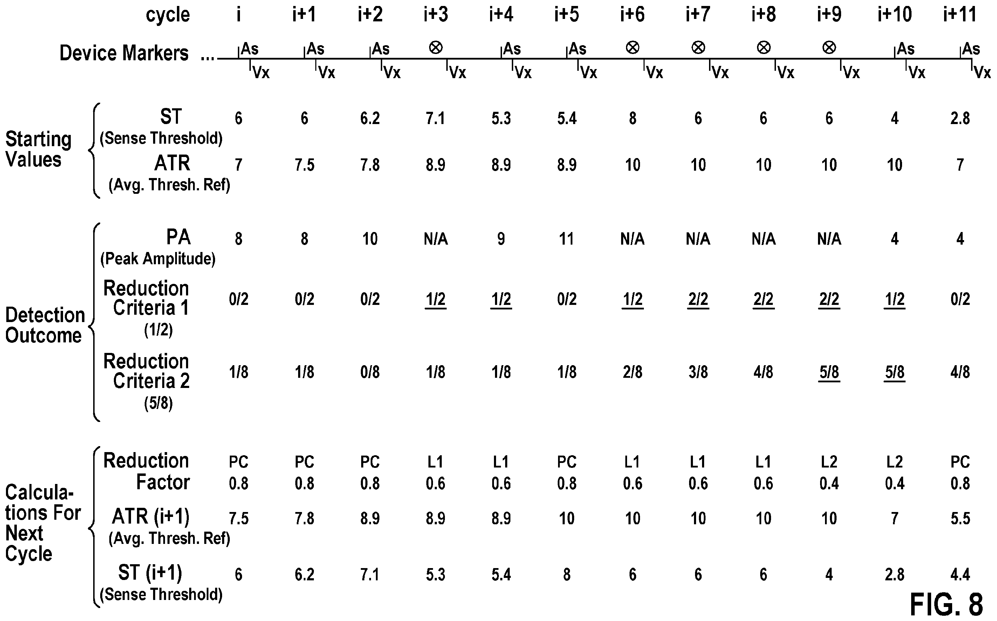

In the example shown in Fig. 7 (and likewise in the example if Fig. 8) in the first row the cardiac cycles are numbered. In the second row detected atrial events As and ventricular events Vx are shown. If no atrial events As are detected, this is indicated by crosses. Also, if a detection occurred, a peak amplitude value (PA) for the measured P-wave in the current cycle is indicated.

In the shown example, the reduction criterion is not fulfilled in cardiac cycles *i* to *i+3.* However, in cardiac cycle *i+4* it is found that in the current and previous cardiac cycles no atrial events have been detected, and correspondingly the "2 out of 2" reduction criterion is fulfilled. Hence, the reduction factor L1 associated with the first reduction criterion is applied, and the sense threshold ST is computed by multiplying the currently valid average threshold reference value ATR (7.5) by the reduction factor L1 (0.45). The threshold ST for cycle i+5 hence assumes a value of 3.4.

In the following cardiac cycles *i+5, i+6* the reduction criterion is not fulfilled, and hence the sense threshold ST is again computed based on the regular percentage ratio PC (0.8). In cardiac cycle *i+7*, however, the reduction criterion again is fulfilled, and the reduction factor L1 is applied to compute the sense threshold ST for the following cycle, i+8.

In the shown example the average threshold reference ATR is computed as ATR(i+1) = W*PA(i) + (1-W)*ATR(i), where the update weight W is 0.5.

In another example, shown in Fig. 8, two reduction criteria are applied. The first criterion is a short-term criterion and is fulfilled if in 1 out of 2 cardiac cycles no atrial events As are detected (sixth row in Fig. 8). The second reduction criterion is a long-term criterion and is fulfilled if in 5 out of 8 cardiac cycles no atrial events As have been detected (seventh row in Fig. 8).

In the example of Fig. 8, no reduction criterion is fulfilled in cardiac cycles *i* to *i+2,* and correspondingly the sense threshold ST is computed by applying the normal percentage ratio PC (0.8). The first reduction criterion is fulfilled in cardiac cycles *i+3* and *i+4,* and correspondingly the reduction factor L1 (0.6) associated with the first reduction criterion is applied to compute the sense threshold ST. In cardiac cycle *i+5* again no reduction criterion is fulfilled, and hence the regular percentage ratio PC is applied. In cardiac cycles *i+6* to *i+8* the first reduction criterion is fulfilled and correspondingly the first reduction factor L1 is applied to compute the sense threshold ST. In cardiac cycle *i+9*, then, also the second reduction criterion is fulfilled, and the reduction factor L2 (0.4) corresponding to the second reduction criterion is applied to compute the sense threshold ST. The second reduction criterion is also fulfilled in cardiac cycle *i+10,* whereas in cardiac cycle *i+11* again no reduction criterion is fulfilled.

By adjusting the sense threshold ST in a step-decay fashion to reduce the sense threshold ST in a step-wise manner in case of missed atrial events As, a reliable atrial capture can be obtained, in particular by suitably lowering the sense threshold ST if in a significant number of cardiac cycles no atrial events have been detected.

Using atrial sense markers As output by the second processing channel 17, ventricular synchronous pacing may be achieved. For this, it can be detected whether, following a detected atrial sense marker As, an intrinsic ventricular sense marker Vx occurs (output by the first processing channel 16) within a predefined time delay window after the atrial sense marker As, in which case no stimulation is required. If no ventricular sense marker Vx is detected, a stimulation pulse may be emitted, causing synchronous pacing in the ventricle.

Conversely, also asynchronous pacing can be performed.

Utilizing a far-field electrical signal received by means of an implantable medical device can offer a superior detection of far-field events, in particular atrial events in case the implantable medical device is implanted into the ventricle. A tracking of far-field events by using and evaluating electrical signals may allow for an increased consistency and reliability in particular with respect to external factors such as posture and patient activity.

The scope of the present invention is defined by the appended claims.

**Claims**

1. An implantable medical device (1) configured to provide for an intracardiac function, the implantable medical device (1) comprising:

   a body (10);
   a sensor arrangement arranged on the body (10) and configured to receive cardiac sense signals; and
   a processing circuitry (15) operatively connected to the sensor arrangement, wherein the processing circuitry (15) is configured to process cardiac sense signals received using the sensor arrangement

   to detect atrial events (As) caused by atrial activity based on a comparison of the cardiac sense signals to a sense threshold (ST) for a number of cardiac cycles,
   to evaluate whether a reduction criterion is fulfilled, and
   to reduce the sense threshold (ST) in a current cycle if in the current cycle it is found that said reduction criterion is fulfilled, wherein the reduction criterion is fulfilled if in X1 out of Y1 cardiac cycles prior to the current cycle no atrial events (As) have been detected, X1 being a natural number equal to or larger than 1 and Y1 being a natural number equal to or larger than X1.

2. The implantable medical device (1) according to claim 1, wherein said sensor arrangement is implemented by an electrode arrangement configured to receive electrical signals as cardiac sense signals.

3. The implantable medical device (1) according to claim 1 or 2, wherein the body (10) is formed by a lead which is connectable to a generator (18) of the implantable medical device (1).

4. The implantable medical device (1) according to claim 1 or 2, wherein the body (10) is formed by a housing of a leadless pacemaker device.

5. The implantable medical device (1) according to one of the preceding claims, wherein the processing circuitry (15) is configured to reduce the sense threshold (ST) by a reduction factor (L1, L2) if said reduction criterion is fulfilled.

6. The implantable medical device (1) according to claim 5, wherein the processing circuitry (15) is configured to reduce the sense threshold (ST) additionally by a step factor (L1, L2) if said reduction criterion is fulfilled for at least two consecutive cycles.

7. The implantable medical device (1) according to one of the preceding claims, wherein the processing circuitry (15) is configured to evaluate whether a further, second reduction criterion is fulfilled, wherein the further, second reduction criterion is fulfilled if in X2 out of Y2 cardiac cycles no atrial events (As) have been detected, X2 being a natural number larger than X1 and Y2 being a natural number larger than Y1.

8. The implantable medical device (1) according to one of the preceding claims, wherein the processing circuitry (15) is configured to reduce the sense threshold (ST) by a second reduction factor (L2) associated with the further, second reduction criterion.

9. The implantable medical device (1) according to one of the preceding claims, wherein the processing circuitry (15) is configured not to reduce the sense threshold (ST) beyond a lower absolute threshold (LAT).

**10.** The implantable medical device (1) according to one of the preceding claims, wherein the processing circuitry (15) is configured to determine a peak amplitude (PA) associated with a detected atrial event (As), wherein the processing circuitry (15) is configured to update the sense threshold (ST) using an average threshold reference based on the equation

$$ST(t) = PC \cdot ATR(t),$$

where ST(t) is the current sense threshold, PC is a percentage ratio, and ATR(t) is the current average threshold reference.

**11.** The implantable medical device (1) according to claim 10, wherein the current average threshold reference is determined by the equation

$$ATR(t) = W \cdot PA(t-1) + (1-W) \cdot ATR(t-1),$$

where W indicates the update weight which determines how much the average threshold reference should change based on the previous peak amplitude..., PA(t-1) is the peak amplitude as determined for the previous cycle t-1, and ATR(t-1) is the previous average threshold reference.

**12.** The implantable medical device (1) according to one of the preceding claims, wherein the processing circuitry (15) comprises a first processing channel (16) having a first gain (G1) for processing a first processing signal derived from cardiac sense signals received via the sensor arrangement and a second processing channel (17) having a second gain (G2) for processing a second processing signal derived from cardiac sense signals received via the sensor arrangement, the second gain (G2) being higher than the first gain (G1).

**13.** The implantable medical device (1) according to claim 12, wherein the processing circuitry (15) is configured to process the first processing signal to detect a ventricular activity and the second processing signal to detect an atrial activity.

**14.** Method for operating an implantable medical device (1) for providing for an intracardiac function, comprising:

receiving, using a sensor arrangement arranged on a body (10) of the implantable medical device (1), cardiac sense signals; and
processing, using a processing circuitry (15) operatively connected to the sensor arrangement, cardiac sense signals received using the sensor arrangement

to detect atrial events (As) caused by atrial activity based on a comparison of the cardiac sense signals to a sense threshold (ST) for a number of cardiac cycles,
to evaluate whether a reduction criterion is fulfilled,, and
to reduce the sense threshold (ST) in the current cycle if in the current cycle it is found that said reduction criterion is fulfilled, wherein the reduction criterion is fulfilled if in X1 out of Y1 cardiac cycles prior to the current cycle no atrial events (As) have been detected, X1 being a natural number equal to or larger than 1 and Y1 being a natural number equal to or larger than X1.

**Patentansprüche**

**1.** Implantierbare medizinische Vorrichtung (1), die dazu konfiguriert ist, eine intrakardiale Funktion bereitzustellen, wobei die implantierbare medizinische Vorrichtung (1) Folgendes umfasst:

einen Körper (10);
eine Sensoranordnung, die an dem Körper (10) angeordnet und dazu konfiguriert ist, kardiale Messsignale zu empfangen; und
eine Verarbeitungsschaltung (15), die mit der Sensoranordnung wirkverbunden ist, wobei die Verarbeitungsschaltung (15) dazu konfiguriert ist, kardiale Messsignale zu verarbeiten, die unter Verwendung der Sensoranordnung empfangen wurden,
um atriale Ereignisse (As), die durch atriale Aktivität verursacht werden, basierend auf einem Vergleich der kardialen Messsignale mit einem Messschwellenwert (ST) für eine Reihe von kardialen Zyklen zu detektieren,
um zu beurteilen, ob ein Reduktionskriterium erfüllt ist, und
um den Messschwellenwert (ST) in einem aktuellen Zyklus zu reduzieren, wenn in dem aktuellen Zyklus festgestellt wird, dass das Reduktionskriterium erfüllt ist, wobei das Reduktionskriterium erfüllt ist, wenn bei X1 von Y1 kardialen Zyklen vor dem aktuellen Zyklus keine atrialen Ereignisse (As) detektiert wurden, wobei X1 eine natürliche Zahl gleich oder größer als 1 ist und Y1 eine natürliche Zahl gleich oder größer als X1 ist.

**2.** Implantierbare medizinische Vorrichtung (1) nach Anspruch 1, wobei die Sensoranordnung von einer Elektrodenanordnung implementiert wird, die dazu konfiguriert ist, elektrische Signale als kardiale Messsignale zu empfangen.

3. Implantierbare medizinische Vorrichtung (1) nach Anspruch 1 oder 2, wobei der Körper (10) von einer Leitung gebildet wird, die mit einem Generator (18) der implantierbaren medizinischen Vorrichtung (1) verbindbar ist.

4. Implantierbare medizinische Vorrichtung (1) nach Anspruch 1 oder 2, wobei der Körper (10) durch ein Gehäuse einer drahtlosen Herzschrittmachervorrichtung gebildet wird.

5. Implantierbare medizinische Vorrichtung (1) nach einem der vorhergehenden Ansprüche, wobei die Verarbeitungsschaltung (15) dazu konfiguriert ist, den Messschwellenwert (ST) um einen Reduktionsfaktor (L1, L2) zu reduzieren, wenn das Reduktionskriterium erfüllt ist.

6. Implantierbare medizinische Vorrichtung (1) nach Anspruch 5, wobei die Verarbeitungsschaltung (15) dazu konfiguriert ist, den Messschwellenwert (St) zusätzlich um einen Schrittfaktor (L1, L2) zu reduzieren, wenn das Reduktionskriterium für mindestens zwei aufeinanderfolgende Zyklen erfüllt ist.

7. Implantierbare medizinische Vorrichtung (1) nach einem der vorhergehenden Ansprüche, wo die Verarbeitungsschaltung (15) dazu konfiguriert ist, zu beurteilen, ob ein weiteres, zweites Reduktionskriterium erfüllt ist, wobei das weitere, zweite Reduktionskriterium erfüllt ist, wenn bei X2 von Y2 kardialen Zyklen keine atrialen Ereignisse (As) detektiert wurden, wobei X2 eine natürliche Zahl größer als X1 ist und Y2 eine natürliche Zahl größer als Y1 ist.

8. Implantierbare medizinische Vorrichtung (1) nach einem der vorhergehenden Ansprüche, wobei die Verarbeitungsschaltung (15) dazu konfiguriert ist, den Messschwellenwert (ST) um einen zweiten Reduktionsfaktor (L2) zu reduzieren, der dem weiteren, zweiten Reduktionskriterium zugeordnet ist.

9. Implantierbare medizinische Vorrichtung (1) nach einem der vorhergehenden Ansprüche, wobei die Verarbeitungsschaltung (15) dazu konfiguriert ist, den Messschwellenwert (ST) nicht über einen niedrigeren absoluten Schwellenwert (LAT) hinaus zu reduzieren.

10. Implantierbare medizinische Vorrichtung (1) nach einem der vorhergehenden Ansprüche, wobei die Verarbeitungsschaltung (15) dazu konfiguriert ist, eine Spitzenamplitude (PA), die dem detektierten atrialen Ereignis (As) zugeordnet ist, zu bestimmen, wobei die Verarbeitungsschaltung (15) dazu konfiguriert ist, den Messschwellenwert (ST) unter Verwendung einer durchschnittlichen Schwellenreferenz basierend auf der folgenden Gleichung zu aktualisieren:

$$ST(t) = PC \cdot ATR(t),$$

wobei ST(t) der aktuelle Messschwellenwert ist, PC ein prozentuales Verhältnis ist und ATR(t) die aktuelle durchschnittliche Schwellenreferenz ist.

11. Implantierbare medizinische Vorrichtung (1) nach Anspruch 10, wobei die aktuelle durchschnittliche Schwellenreferenz durch die folgende Gleichung bestimmt wird:

$$ATR(t) = W \cdot PA(t-1) + (1-W) \cdot ATR(t-1),$$

wobei W die aktualisierte Gewichtung angibt, die bestimmt, um welchen Betrag sich die durchschnittliche Schwellenreferenz basierend auf der vorherigen Spitzenamplitude ändern sollte, PA(t-1) die Spitzenamplitude ist, wie für den vorherigen Zyklus t-1 bestimmt, und ATR(t-1) die vorherige durchschnittliche Schwellenreferenz ist.

12. Implantierbare medizinische Vorrichtung (1) nach einem der vorhergehenden Ansprüche, wobei die Verarbeitungsschaltung (15) einen ersten Verarbeitungskanal (16) mit einer ersten Verstärkung (G1) zum Verarbeiten eines ersten Verarbeitungssignals, das von kardialen Messsignalen abgeleitet wurde, die über die Sensoranordnung empfangen wurden, und einen zweiten Verarbeitungskanal (17) mit einer zweiten Verstärkung (G2) zum Verarbeiten eines zweiten Verarbeitungssignals, das von kardialen Messsignalen abgeleitet wurde, die über die Sensoranordnung empfangen wurden, umfasst, wobei die zweite Verstärkung (G2) größer als die erste Verstärkung (G1) ist.

13. Implantierbare medizinische Vorrichtung (1) nach Anspruch 12, wobei die Verarbeitungsschaltung (15) dazu konfiguriert ist, das erste Verarbeitungssignal zu verarbeiten, um eine ventrikuläre Aktivität zu detektieren, und das zweite Verarbeitungssignal zu verarbeiten, um eine atriale Aktivität zu detektieren.

14. Verfahren zum Betreiben einer implantierbaren medizinischen Vorrichtung (1) zum Bereitstellen einer intrakardialen Funktion, umfassend:

   Empfangen, unter Verwendung einer Sensoranordnung, die an einem Körper (10) der implantierbaren medizinischen Vorrichtung (1) angeordnet ist, von kardialen Messsignalen; und Verarbeiten, unter Verwendung einer Verarbeitungsschaltung (15), die mit der Sensoranordnung wirkverbunden ist, von kardialen Messsignalen, die unter Verwendung der Sensoranordnung empfangen wurden,

um atriale Ereignisse (As), die durch atriale Aktivität verursacht werden, basierend auf einem Vergleich der kardialen Messsignale mit einem Messschwellenwert (ST) für eine Reihe von kardialen Zyklen zu detektieren, um zu beurteilen, ob ein Reduktionskriterium erfüllt ist, und

um den Messschwellenwert (ST) in dem aktuellen Zyklus zu reduzieren, wenn in dem aktuellen Zyklus festgestellt wird, dass das Reduktionskriterium erfüllt ist, wobei das Reduktionskriterium erfüllt ist, wenn bei X1 von Y1 kardialen Zyklen vor dem aktuellen Zyklus keine atrialen Ereignisse (As) detektiert wurden, wobei X1 eine natürliche Zahl gleich oder größer als 1 ist und Y1 eine natürliche Zahl gleich oder größer als X1 ist.

## Revendications

1. Dispositif médical implantable (1) conçu pour pourvoir à une fonction intracardiaque, le dispositif médical implantable (1) comprenant :

un corps (10) ;
un agencement de capteurs agencé sur le corps (10) et configuré pour recevoir des signaux de détection cardiaque ; et
un circuit de traitement (15) connecté de manière fonctionnelle à l'agencement de capteurs, dans lequel le circuit de traitement (15) est configuré pour traiter les signaux de détection cardiaque reçus en utilisant l'agencement de capteurs

pour détecter des événements auriculaires (As) provoqués par une activité auriculaire en se basant sur une comparaison des signaux de détection cardiaque avec un seuil de détection (ST) pour un nombre de cycles cardiaques,
pour évaluer si un critère de réduction est rempli, et
pour réduire le seuil de détection (ST) dans un cycle actuel si dans le cycle actuel il est découvert que ledit critère de réduction est rempli, dans lequel le critère de réduction est rempli si dans X1 cycles cardiaques sur Y1 avant le cycle actuel aucun événement auriculaire (As) n'a été détecté, X1 étant un entier naturel supérieur ou égal à 1 et Y1 étant un entier naturel supérieur ou égal à X1.

2. Dispositif médical implantable (1) selon la revendication 1, dans lequel ledit agencement de capteurs est mis en œuvre par un agencement d'électrodes configuré pour recevoir des signaux électriques en tant que signaux de détection cardiaque.

3. Dispositif médical implantable (1) selon la revendication 1 ou 2, dans lequel le corps (10) est formé par un câble qui peut être connecté à un générateur (18) du dispositif médical implantable (1).

4. Dispositif médical implantable (1) selon la revendication 1 ou 2, dans lequel le corps (10) est formé par un boîtier d'un dispositif de stimulateur cardiaque sans câbles.

5. Dispositif médical implantable (1) selon l'une des revendications précédentes, dans lequel le circuit de traitement (15) est configuré pour réduire le seuil de détection (ST) d'un facteur de réduction (L1, L2) si ledit critère de réduction est rempli.

6. Dispositif médical implantable (1) selon la revendication 5, dans lequel le circuit de traitement (15) est configuré pour réduire le seuil de détection (ST) en outre par un facteur d'incrémentation (L1, L2) si ledit critère de réduction est rempli pendant au moins deux cycles consécutifs.

7. Dispositif médical implantable (1) selon l'une des revendications précédentes, dans lequel le circuit de traitement (15) est configuré pour évaluer si un second critère de réduction supplémentaire est rempli, dans lequel le second critère de réduction supplémentaire est rempli si dans X2 cycles cardiaques sur Y2 aucun événement auriculaire (As) n'a été détecté, X2 étant un entier naturel supérieur à X1 et Y2 étant un entier naturel supérieur à Y1.

8. Dispositif médical implantable (1) selon l'une des revendications précédentes, dans lequel le circuit de traitement (15) est configuré pour réduire le seuil de détection (ST) d'un second facteur de réduction (L2) associé au second critère de réduction supplémentaire.

9. Dispositif médical implantable (1) selon l'une des revendications précédentes, dans lequel le circuit de traitement (15) est configuré pour ne pas réduire le seuil de détection (ST) au-delà d'un seuil inférieur absolu, ou LAT.

10. Dispositif médical implantable (1) selon l'une des revendications précédentes, dans lequel le circuit de traitement (15) est configuré pour déterminer une amplitude de pic (PA) associée à un événement auriculaire (As) détecté, dans lequel le circuit de traitement (15) est configuré pour mettre à jour le seuil de détection (ST) en utilisant une référence de seuil moyenne basée sur l'équation

$$ST(t) = PC \cdot ATR(t),$$

où ST(t) est le seuil de détection actuel, PC est un rapport de pourcentage, et ATR(t) est la référence de seuil moyen actuelle.

11. Dispositif médical implantable (1) selon la revendication 10, dans lequel la référence de seuil moyen actuelle est déterminée par l'équation

$$ATR(t) = W \cdot PA(t-1) + (1-W) \cdot ATR(t-1),$$

où W indique la pondération mise à jour qui détermine la proportion dont la référence de seuil moyen devrait changer en se basant sur l'amplitude de pic précédente..., PA(t-1) est l'amplitude de pic comme déterminé pour le cycle précédent t-1, et ATR(t-1) est la référence de seuil moyen précédente.

12. Dispositif médical implantable (1) selon l'une des revendications précédentes, dans lequel le circuit de traitement (15) comprend un premier canal de traitement (16) ayant un premier gain (G1) pour traiter un premier signal de traitement dérivé de signaux de détection cardiaque reçus par l'intermédiaire de l'agencement de capteurs et un second canal de traitement (17) ayant un second gain (G2) pour traiter un second signal de traitement dérivé de signaux de détection cardiaque reçus par l'intermédiaire de l'agencement de capteurs, le second gain (G2) étant supérieur au premier gain (G1).

13. Dispositif médical implantable (1) selon la revendication 12, dans lequel le circuit de traitement (15) est conçu pour traiter le premier signal de traitement afin de détecter une activité ventriculaire et le second signal de traitement afin de détecter une activité auriculaire.

14. Procédé de fonctionnement d'un dispositif médical implantable (1) destiné à fournir une fonction intracardiaque, comprenant les étapes consistant à :

recevoir, en utilisant un agencement de capteur agencé sur un corps (10) du dispositif médical implantable (1), des signaux de détection cardiaque ; et
traiter, en utilisant un circuit de traitement (15) connecté de manière fonctionnelle à l'agencement de capteurs, les signaux de détection cardiaque reçus en utilisant l'agencement de capteurs

pour détecter des événements auriculaires (As) provoqués par une activité auriculaire en se basant sur une comparaison des signaux de détection cardiaque avec un seuil de détection (ST) pour un nombre de cycles cardiaques,
pour évaluer si un critère de réduction est rempli, et
pour réduire le seuil de détection (ST) dans le cycle actuel si dans le cycle actuel il est découvert que ledit critère de réduction est rempli, dans lequel le critère de réduction est rempli si dans X1 cycles cardiaques sur Y1 avant le cycle actuel aucun événement auriculaire (As) n'a été détecté, X1 étant un entier naturel supérieur ou égal à 1 et Y1 étant un entier naturel supérieur ou égal à X1.

**FIG. 1**

FIG. 3

FIG. 2

FIG. 4

EP 4 294 506 B1

FIG. 5A

FIG. 5B

FIG. 6

EP 4 294 506 B1

EP 4 294 506 B1

| cycle | i | i+1 | i+2 | i+3 | i+4 | i+5 | i+6 | i+7 | i+8 | i+9 |
|---|---|---|---|---|---|---|---|---|---|---|
| Device Markers ... | As Vx | As Vx | As Vx | ⊗ Vx | ⊗ Vx | As Vx | ⊗ Vx | ⊗ Vx | As Vx | ⊗ Vx |
| **Starting Values** | | | | | | | | | | |
| ST (Sense Threshold) | 6 | 6.4 | 6.4 | 6 | 6 | 3.4 | 4.6 | 4.6 | 2.6 | 4.3 |
| ATR (Avg. Thresh. Ref) | 7 | 8 | 8 | 7.5 | 7.5 | 7.5 | 5.8 | 5.8 | 5.8 | 5.4 |
| **Detection Outcome** | | | | | | | | | | |
| PA (Peak Amplitude) | 9 | 8 | 7 | N/A | N/A | 4 | N/A | N/A | 5 | N/A |
| Reduction Criteria 1 (2/2) | 0/2 | 0/2 | 0/2 | 1/2 | 2/2 | 1/2 | 1/2 | 2/2 | 1/2 | 1/2 |
| **Calculations For Next Cycle** | | | | | | | | | | |
| Reduction Factor | PC 0.8 | PC 0.8 | PC 0.8 | PC 0.8 | L1 0.45 | PC 0.8 | PC 0.8 | L1 0.45 | PC 0.8 | PC 0.8 |
| ATR (i+1) (Avg. Thresh. Ref) | 8 | 8 | 7.5 | 7.5 | 7.5 | 5.8 | 5.8 | 5.4 | 5.4 | 5.4 |
| ST (i+1) (Sense Threshold) | 6.4 | 6.4 | 6 | 6 | 3.4 | 4.6 | 4.6 | 2.6 | 4.3 | 4.3 |

FIG. 7

| | cycle i | i+1 | i+2 | i+3 | i+4 | i+5 | i+6 | i+7 | i+8 | i+9 | i+10 | i+11 |
|---|---|---|---|---|---|---|---|---|---|---|---|---|
| Device Markers ... | As / Vx | As / Vx | As / Vx | ⊗ / Vx | As / Vx | As / Vx | ⊗ / Vx | ⊗ / Vx | ⊗ / Vx | ⊗ / Vx | As / Vx | As / Vx |
| **Starting Values** | | | | | | | | | | | | |
| ST (Sense Threshold) | 6 | 6 | 6.2 | 7.1 | 5.3 | 5.4 | 8 | 6 | 6 | 6 | 4 | 2.8 |
| ATR (Avg. Thresh. Ref) | 7 | 7.5 | 7.8 | 8.9 | 8.9 | 8.9 | 10 | 10 | 10 | 10 | 10 | 7 |
| **Detection Outcome** | | | | | | | | | | | | |
| PA (Peak Amplitude) | 8 | 8 | 10 | N/A | 9 | 11 | N/A | N/A | N/A | N/A | 4 | 4 |
| Reduction Criteria 1 (1/2) | 0/2 | 0/2 | 0/2 | 1/2 | 1/2 | 0/2 | 1/2 | 2/2 | 2/2 | 2/2 | 1/2 | 0/2 |
| Reduction Criteria 2 (5/8) | 1/8 | 1/8 | 0/8 | 1/8 | 1/8 | 1/8 | 2/8 | 3/8 | 4/8 | 5/8 | 5/8 | 4/8 |
| **Calculations For Next Cycle** | | | | | | | | | | | | |
| Reduction Factor | PC 0.8 | PC 0.8 | PC 0.8 | L1 0.6 | L1 0.6 | PC 0.8 | L1 0.6 | L1 0.6 | L1 0.6 | L2 0.4 | L2 0.4 | PC 0.8 |
| ATR (i+1) (Avg. Thresh. Ref) | 7.5 | 7.8 | 8.9 | 8.9 | 8.9 | 10 | 10 | 10 | 10 | 10 | 7 | 5.5 |
| ST (i+1) (Sense Threshold) | 6 | 6.2 | 7.1 | 5.3 | 5.4 | 8 | 6 | 6 | 6 | 4 | 2.8 | 4.4 |

FIG. 8

EP 4 294 506 B1

**FIG. 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180021581 A1
- US 20080262559 A1

- EP 2108400 A1